# EUROPEAN PATENT SPECIFICATION

(11) **EP 2 949 348 B1**
(45) Date of publication and mention of the grant of the patent: **15.11.2017**
(21) Application number: 14743521.8
(22) Date of filing: 22.01.2014
(51) Int. Cl.: A61L 27/00, A01K 67/027, A61K 35/407, A61L 15/16

(54) **ADHESION PREVENTING MATERIAL**
HAFTVERHINDERNDES MATERIAL
MATÉRIAU DE PRÉVENTION D'ADHÉRENCE

(30) Priority: 22.01.2013 US 201361755163 P
(43) Date of publication of application: 02.12.2015
(73) Proprietor: The University of Tokyo, Tokyo 113-8654 (JP)
(72) Inventor: MIYAJIMA, Atsushi, Tokyo 113-8654 (JP); INAGAKI, Natsuko, Tokyo 113-8654 (JP); INAGAKI, Fuyuki, Tokyo 113-8654 (JP); KOKUDO, Norihiro, Tokyo 113-8654 (JP)
(74) Representative: Keirstead, Tanis Evelyne
(86) International application number: PCT/JP2014/051280
(87) International publication number: WO 2014/115776

(56) References cited:
- WO-A1-2005/047496
- JP-A- 2005 000 608
- Ryoji Takazawa: "Mesothelial cell sheets cultured on fibrin gel prevent adhesion formation inan intestinal hernia model", , 1 March 2005 (2005-03-01), XP055280522, Retrieved from the Internet: URL:http://online.liebertpub.com/doi/pdf/1 0.1089/ten.2005.11.618 [retrieved on 2016-06-15]
- M T R Yamato Asano Takazawa ET AL: "Transplantation of an autologous mesothelial cell sheet prepared from tunica vaginalis prevents post-operative adhesions in a canine model", Pain Digest, 1 November 2006 (2006-11-01), page 289, XP055280501, Retrieved from the Internet: URL:http://online.liebertpub.com/doi/pdf/1 0.1089/ten.2006.12.2629
- RYOICHI SAKIYAMA: "Manufacture of Mesothelial Cell Sheet Using Temperature Responsive Culture Dish", MAKU - MEMBRANE, KITAMI SHOBO, TOKYO, JP, vol. 37, no. 3, 1 January 2012 (2012-01-01), pages 108-112, XP008180226, ISSN: 0385-1036
- DATABASE WPI Week 200506 Thomson Scientific, London, GB; AN 2005-052397 XP002758799, & JP 2005 000608 A (OKANO M) 6 January 2005 (2005-01-06)
- ONITSUKA I ET AL: "Characterization and Functional Analyses of Hepatic Mesothelial Cells in Mouse Liver Development", GASTROENTEROLOGY, ELSEVIER, AMSTERDAM, NL, vol. 138, no. 4, 1 April 2010 (2010-04-01) , pages 1525-1535.e6, XP026985010, ISSN: 0016-5085 [retrieved on 2010-01-18]
- KUNIO KAWANISHI ET AL. FUKUMAKU SAIBO SHEET NO KAIHATSU TO JUTSUGO CHOKAN YUCHAKU MODEL NI OKERU YUCHAKU BOSHI KOKA NO KENTO vol. 32, 2012, TOKYO, pages 72 - 73, XP008180253
- KUNIO KAWANISHI ET AL.: 'Fukumaku Saibo Sheet no Kaihatsu to Jutsugo Chokan Yuchaku Model ni Okeru Yuchaku Boshi Koka no Kento' SAISEI IRYO vol. 11, 2012, page 199, XP008180249
- RYOICHI SAKIYAMA: 'Manufacture of Mesothelial Cell Sheet Using Temperature Responsive Culture Dish' MEMBRANE vol. 37, no. 3, 2012, pages 108 - 112, XP008180226
- RYOICHI SAKIYAMA ET AL.: 'Chuhi Saibo Sheet ni yoru Fukumaku Shogai Kaizen eno Kokoromi' THERAPEUTICS & ENGINEERING vol. 24, no. 1, 2012, pages 45 - 48, XP008180259
- ONITSUKA, I. ET AL.: 'Characterization and Functional Analyses of Hepatic Mesothelial Cells in Mouse Liver Development.' GASTROENTEROLOGY vol. 138, no. 4, 2010, pages 1525 - 1535, XP026985010
- TAKASHI SUHARA ET AL.: 'Kanzo Setsujo ni Okeru in situ Kakyo Hyaluronic Acid Hydrogel Yuchaku Boshizai no Kaihatsu' JAPANESE SOCIETY FOR BIOMATERIALS SYMPOSIUM 2012 YOKOSHU 2012, page 221, XP008180250
- RYOICHI SAKIYAMA ET AL.: 'Fukumaku Sen'isho Kaizen no Tameno Hito Chuhi Saibokabu Saibo Sheet no Kaihatsu' JOURNAL OF JAPANESE SOCIETY FOR DIALYSIS THERAPY vol. 40, no. 1, 2007, page 307, XP008180252
- RYOICHI SAKIYAMA ET AL.: 'Hito Chuhi Saibo Sheet ni yoru Shogai Fukumaku Kaizen no Koka' JOURNAL OF JAPANESE SOCIETY FOR DIALYSIS THERAPY vol. 44, no. 1, 2011, page 406, XP008180338
- TARO UYAMA: 'Kokino Saibo Baiyo Kizai ' Cell ' Series' GEKKAN MEDICAL SCIENCE DIGEST vol. 33, no. 9, 2007, pages 1029 - 1033, XP008180257
- NORIKO AJIRO: 'Saibo Sheet Kaishuyo Ondo Otosei Saibo Baiyo Kizai 'UpCell' BIO CLINICA vol. 23, no. 7, 2008, pages 649 - 650, XP008180248

### SEQUENCE LISTING

<110> The University of Tokyo
<120> ADHESION PREVENTING MATERIAL
<130> TEK/FP7146145
<140> EP14743521.8
   <141> 2014-01-22
<150> PCT/JP2014/051280
   <151> 2014-01-22
<150> US 61/755,163
   <151> 2013-01-22
<160> 12
<170> PatentIn version 3.1
<210> 1
   <211> 18
   <212> DNA
   <213> Mus musculus
<400> 1
   tccttgttgc tgccctct 18
<210> 2
   <211> 18
   <212> DNA
   <213> Mus musculus
<400> 2
   ctctgtgagc cgttgctg 18
<210> 3
   <211> 20
   <212> DNA
   <213> Mus musculus
<400> 3
   caccccttgg gagtattgtg 20
<210> 4
   <211> 22
   <212> DNA
   <213> Mus musculus
<400> 4
   gggacatcag tctcattcac ag 22
<210> 5
   <211> 25
   <212> DNA
   <213> Mus musculus
<400> 5
   gctaccaaac tggatataat cagga 25
<210> 6
   <211> 24
   <212> DNA
   <213> Mus musculus
<400> 6
   ccaggtagct atggtactcc agaa 24
<210> 7
   <211> 20
   <212> DNA
   <213> Mus musculus
<400> 7
   cgcactggta aaaccgaact 20
<210> 8
   <211> 18
   <212> DNA
   <213> Mus musculus
<400> 8
   gaagaagcct cggtgctg 18
<210> 9
   <211> 19
   <212> DNA
   <213> Mus musculus
<400> 9
   aggacctctg caagccaaa 19
<210> 10
   <211> 21
   <212> DNA
   <213> Mus musculus
<400> 10
   cacagctgcc aagatgaaaa t 21
<210> 11
   <211> 20
   <212> DNA
   <213> Mus musculus
<400> 11
   ctaaggccaa ccgtgaaaag 20
<210> 12
   <211> 20
   <212> DNA
   <213> Mus musculus
<400> 12
   accagaggca tacagggaca 20

## Description

### Technical Field

The present invention relates to an adhesion preventing material consisting of a mesothelial cell sheet, and the like.

### Background Art

In the context of an increase in the number of patients suffering from non-alcoholic steatohepatitis (NASH) or colon cancer patients due to westernization of lifestyle, the number of patients suffering from primary/metastatic liver cancer is expected to increase in future.

As a treatment method for liver cancer which has a high recurrence rate, "repeated hepatectomy" has been accepted as one of the most effective methods which can extend the survival term or from which radical cure can be expected and it has been performed widely. The repeated hepatectomy however has a problem of postoperative adhesion. When postoperative adhesion occurs, adhesiotomy becomes necessary before hepatectomy is performed again. This increases the risk of prolongation of operation time, increase in blood loss, and increase in the rate of complications such as infections.

Adhesion is a phenomenon inevitably associated with abdominal surgeries and it occurs irrespective of age, sex, or race. It occurs not only in the liver but also in various places and may cause ileus, chronic pelvic pain, stomachache, sterility, or the like.

For postoperative adhesion which is said to occur at a rate as high as from 93 to 100% after surgical operations (including not so serious cases), various treatment or prevention methods have so far been proposed. Examples of them include separation of adhesion by reoperation through laparotomy or laparoscopic surgery. However, the former one places an excessive burden on patients, while the latter one requires operators to have advanced skill. In addition, they may cause adhesion again due to the reoperation.

A method of reducing postoperative adhesion by administration of pharmacological agents has been attempted, but effect of it is hardly recognized at present.

One of the currently most common methods for treating postoperative adhesion is placing an absorptive adhesion preventing material made of a polymer material. It is reported that placing an absorptive adhesion preventing material reduces the extent of adhesion (Non-patent Document 1). However, it has been elucidated from the results of meta-analysis in recent years that the above method does not decrease the frequency of ileus and increases the frequency of intra-abdominal abscess and anastomotic leaks (Non-patent Documents 2 and 3). Further, it has been pointed out that using an absorptive adhesion preventing agent may increase the possibility of inhibiting wound healing or, when it is used for the liver, may increase the risk of postoperative bile leakage.

Non-Patent Document 4 discloses mesothelial cell sheets obtained by enzymatic disaggregation of mesentery cultured on fibrin gel for preventing adhesion formation in an intestinal hernia model.

Non-Patent Document 4 also teaches that in clinical cases autologous mesothelial cells can be isolated and can be used to repair the wounded peritoneum to reduce post-operative complications caused by adhesive formation.

Non-Patent Document D5 discloses the transplantation of an autologous mesothelial cell sheet prepared from tunica vaginal is to prevent post-operative peritoneal adhesions in a canin model and its clinical application.

Non-Patent Document D6 discloses the manufacture of a mesothelial cell sheet and its application to repair damaged peritoneum.

JP 2005 000608 discloses a cell sheet containing autologous mesothelial cells for use in regenerative therapy of peritoneum affected by an injury or defect and shows that the adhesion was prevented.

In repeated hepatectomy, there occurs a problem of deterioration in functional reserve of the remnant liver in addition to the problem of adhesion. The deterioration in functional reserve may lead to liver failure so that it inevitably limits the frequency or range of repeated hepatectomy.

### Citation List

### Non-Patent Documents

Non-Patent Document 1: Fazio, VW. et al., Dis Colon Rectum, 49, 1-11, 2006
Non-Patent Document 2: Tang, CL. et al., Ann Surg., 243, 449-455, 2006
Non-Patent Document 3: Zeng, Q. et al., World J Surg., 31, 2125-2131, 2007
Non-patent Document 4: Takazawa et al., Tissue engineering, Vol.11, Number 3/4, 2005, p.618-625
Non-patent Document 5: Takazawa et al., Tissue engineering, Vol.12, Number 9, 2006, p.2629-2637
Non-patent Document 6: Sakiyama, Membrane 37(3), 108-112 (2012)

### Disclosure of the Invention

### Problem to be Solved by the Invention

An object of the present invention is to provide a material for use in a method of preventing adhesion of a resected surface after partial hepatectomy, wherein the adhesion preventing material is capable of preventing adhesion safely and efficiently.

### Means for Solving the Problem

The present inventors have proceeded with an extensive investigation in order to overcome the above-mentioned problems. As a result, it has been found that adhesion can be prevented safely by using a cell sheet prepared using mesothelial cells as an adhesion preventing material.

It has also been found that an adhesion preventing material prepared using liver mesothelial cells, when applied to a resected surface of the liver that has been subjected to partial hepatectomy, prevents adhesion and at the same time, promotes liver regeneration.

In addition, it has been confirmed that even a mesothelial cell sheet prepared using cells derived from an individual other than a recipient can function, leading to the completion of the present invention.

The present invention relates to:
[1] A material for use in a method of preventing adhesion of a resected surface after partial hepatectomy, wherein the material comprises a cell sheet comprising liver mesothelial cells.
[2] the adhesion preventing material for use as described above in [1], wherein the mesothelial cells are cells derived from the same individual as the subject of the adhesion prevention;
[3] the adhesion preventing material for use as described above in [1], wherein the mesothelial cells are cells derived from an individual different from the subject of the adhesion prevention;
[4] the adhesion preventing material for use as described above in any one of [1] to [3], wherein the mesothelial cells are cells derived from an individual from fetal to infant stages;
[5] the material for use according to any one of [1] to [4] above, wherein the material is placed on the resected surface;
[6] the material for use according to any one of [1] to [5] above, wherein the material is further for use in promoting liver regeneration;
[7] the adhesion preventing material as described above in any one of [1] to [6], which does not contain a support but contains only the cell sheet.

### Effect of the Invention

The adhesion preventing material for use in the present invention is comprised of mesothelial cells which are biomaterials so that it can be applied to the organ or tissue safely and can prevent adhesion without inhibiting wound healing.

It can also prevent adhesion of a resected surface after partial hepatectomy, though conventional methods cannot. A cell sheet using liver mesothelial cells can prevent adhesion and at the same time promote liver regeneration after partial hepatectomy so that it may suppress postoperative liver failure and increase the frequency of repeated liver transplantation.

The adhesion preventing material for use in the present invention can function even when it is prepared using cells derived from an individual other than the recipient so that it is useful for clinical application.

### Brief Description of the Drawings

[FIG. 1] FIG. 1 schematically shows an adhesion preventing method using an adhesion preventing material according to the present invention.
[FIG. 2] The left panels of FIG. 2 show photographs of a PPHx mouse model onto which a liver mesothelial cell sheet was autotransplanted and the other model onto which no sheet was transplanted, each on postoperative day 10. The right panel of FIG.2 shows the results of autotransplanting a liver mesothelial cell sheet, a peritoneal mesothelial cell sheet, and a fibroblast cell sheet onto the liver resected surface of PPHx mouse models, respectively, and calculating an adhesion percentage on postoperative day 10.
[FIG. 3] FIG. 3 shows the results of evaluating postoperative liver regeneration in the liver mesothelial cell transplantation group, the peritoneal mesothelial cell transplantation group, and the non-transplantation group with a percentage of Ki67 positive hepatocytes as an indicator.
[FIG. 4] FIG. 4 shows the results of evaluating postoperative liver regeneration in the liver mesothelial cell transplantation group, the peritoneal mesothelial cell transplantation group, and the non-transplantation group with the serum albumin as an indicator.
[FIG. 5] FIG. 5 shows the results of transplanting a cell sheet using the liver mesothelial cells derived from a C57BL/6J mouse onto a BALB/c PPHx mouse model and determining an adhesion percentage.
[FIG. 6] FIG. 6 shows the results of transplanting a cell sheet using the liver mesothelial cells derived from a C57BL/6J mouse onto a BALB/c PPHx mouse model and evaluating the postoperative liver regeneration with a percentage of Ki67 positive hepatocytes as an indicator.
[FIG. 7] FIG. 7 shows the observation results of a peritoneal adhesion mouse model which was transplanted with the cell sheet using the liver mesothelial cells and was then subjected to laparotomy 10 days after transplantation.
[FIG. 8] FIG. 8 shows the observation results of a peritoneal adhesion mouse model which was subjected to abdominal closure without transplanting a cell sheet and after 10 days, was subjected to laparotomy.

### Embodiment for Carrying out the Invention

The adhesion preventing material for use in the present invention is consisting of a cell sheet containing liver mesothelial cells.

The term "adhesion preventing material" as used herein means a cell sheet to be transplanted, for preventing "adhesion" which is a phenomenon that originally separated organs or tissues bond to each other, onto the organs, tissues, or the like which may cause adhesion.

The term "preventing" as used herein means not only preventing adhesion completely but also significantly suppressing adhesion.

The term "cell sheet" as used herein means a sheet containing cells as a main component. Although it is not particularly limited insofar as it can be attached to the organ or tissue, it may be a monolayer cell sheet, a sheet comprised of two or more layers of cells, or a sheet comprised of cells obtained by three-dimensional culture. The cell sheet is typically a monolayer cell sheet. The cell sheet may contain an extracellular matrix.

The term "mesothelial cells" as used herein means monolayer cells that cover the uppermost surface of the mesothelium. The mesothelium is a pattern tissue that covers the surface of body cavities such as thoracic cavity, pericardium, and abdominal cavity.

In isolating mesothelial cells from the tissues or organs, they may be derived from individuals of any stage from fetus to adult. For example, they can be obtained from individuals from fetal to infant stages, that is, individuals from a fetal stage to a stage of a child less than about six years old. Cells derived from individuals from fetal to infant stages abundantly contain growth factors such as hepatocyte growth factor (HGF) and epidermal growth factor (EGF) so that they are useful not only for adhesion prevention but also for regeneration promotion of the organs or tissues. The term "regeneration promotion of the organs or tissues" as used herein means restoration, to its original state, of the entirety or portion of the constitution or function which the organs or tissues originally have but is damaged by surgery or adhesion.

In addition, the mesothelial cells may be derived from the same individual as the recipient to be transplanted with the adhesion preventing material or may be derived from an individual different from the recipient.

In one embodiment, the "adhesion preventing material" may contain a support necessary for cell culture or for maintaining the shape of the cell sheet. In another embodiment, the "adhesion preventing material" may be substantially consisting only of the cell sheet. The adhesion preventing material substantially consisting only of the cell sheet has an advantage that it easily achieves a function of promoting regeneration of the organs or tissues, because it can reproduce a state closer to nature in the living body.

The "cell sheet comprising mesothelial cells" as used herein may contain other cells insofar as it contains mesothelial cells. The proportion of mesothelial cells in the total number of cells contained in the cell sheet may be, for example, 50% or more, 60% or more, 70% or more, 80% or more, 90% or more, 95% or more, 98% or more, or 99% or more. The mesothelial cells may contain mesothelial precursor cells which will differentiate into mesothelial cells.

In the present invention, the mesothelial cells are liver mesothelial cells. The liver mesothelial cells are mesothelial cells that cover the surface of the liver. The adhesion preventing material consisting of a cell sheet comprising the liver mesothelial cells is useful for adhesion prevention of a resected surface after partial hepatectomy. The intraperitoneal adipose tissue or the like is easy to attach to the resected surface after partial hepatectomy and cause adhesion.

For the treatment of primary liver cancer or colorectal cancer liver metastasis, an active attempt to perform "repeated hepatectomy" to prevent recurrence in the remnant liver after hepatectomy is only one curative treatment from which radical cure or prolongation of life can be expected. When adhesion occurs in such a case, it must be separated during second or third surgery, which increases the risk of massive hemorrhage or infection. In addition, the adhesion deteriorates hepatic functional reserve so that it limits the frequency of hepatectomy and at the same time, it is likely to cause postoperative liver failure.

The adhesion preventing material consisting of a cell sheet comprising liver mesothelial cells can significantly prevent adhesion of the resected surface after partial hepatectomy and further, promote liver regeneration, thereby preventing deterioration in thepatic functional reserve.

The term "resected surface" as used herein means a surface which appears after resection or separation of a portion of the organ or tissue.

The term "hepatic functional reserve" as used herein means the function of the liver itself. Hepatic functional reserve can be evaluated by a known method. For example, liver damage classification or Child-Pugh classification is frequently used for the evaluation.

In the liver damage classification, hepatic functional reserve is evaluated by measuring ascites, serum bilirubin, serum albumin, ICG R15, and prothrombin activity, while in the Child-Pugh, it is evaluated by measuring encephalopathy, ascites, serum bilirubin, serum albumin, and prothrombin activity.

The term "promoting liver regeneration" as used herein means achieving at least one of the following: improvement in at least one of the measurement values of ascites, serum bilirubin, serum albumin, ICG R15, prothrombin activity, and encephalopathy, improvement in evaluation by the liver damage classification or Child-Pugh classification, increase in the size of the liver, increase in the weight of the liver, promotion of proliferation of hepatocytes, and increase in the size of hepatocytes.

When the adhesion preventing material of the present invention contains an extracellular matrix therein, it does not need suturing and it can be engrafted onto the surface or resected surface of an organ or tissue only by placing it thereto.

The adhesion preventing material of the present invention can be prepared by a known method for preparing a cultured cell sheet or a method similar thereto.

As one example, a cell sheet can be obtained by culturing mesothelial cells in a culture dish coated with a temperature responsive polymer, changing the temperature when the mesothelial cells form a sheet, and collecting the resulting sheet from the culture dish. Examples of the temperature responsive polymer include polymers which become hydrophobic from hydrophilic by a temperature change. As the temperature responsive polymer, known ones and those similar thereto can be used. A commercially available culture dish coated in advance with a temperature responsive polymer may be used. By using the culture dish coated with a temperature responsive polymer, an adhesion preventing material that does not contain a support but is substantially consisting only of a mesothelial cell sheet can be obtained.

In one embodiment, the method of promoting liver regeneration according to the present invention includes a step of placing a cell sheet comprising liver mesothelial cells on a resected surface after partial hepatectomy. This method can prevent adhesion, which could become a problem during repeated hepatectomy and at the same time promote recovery of hepatic functional reserve so that it becomes possible to prevent postoperative liver failure and increase the frequency of hepatectomy.

In one embodiment, the method of preparing a partial hepatectomy adhesion rodent model as described herein includes a step of tying and cutting the median lobe and a step of cutting the middle part of the left lobe.

The liver of the rodent, different from the human liver, is separated into a left lobe, a median lobe, a right lobe, and a caudate lobe and each lobe is in cluster form. The entirety of each of the lobes is therefore resected from the conventional mouse hepatectomy model so that an adhesion model having a liver resected surface cannot be obtained.

As described herein, on the other hand, the liver resected surface can be formed in the left lobe so that a rodent model having adhesion thereon can be obtained.

The method of preparing a partial hepatectomy adhesion rodent model as described herein may further include a step of placing the epididymis adipose tissue in the vicinity of the resected surface of the left lobe. Placement of the adipose tissue in the vicinity of the resected surface promotes attachment of the adipose tissue thereto, facilitating the occurrence of adhesion.

A partial hepatectomy adhesion rodent model as described herein has a resected surface in the left lobe of the liver.

### Examples

The present invention will hereinafter be described specifically based on Examples, but the present invention is not limited to or by them.

### [Materials and methods]

### 1. Outline of method

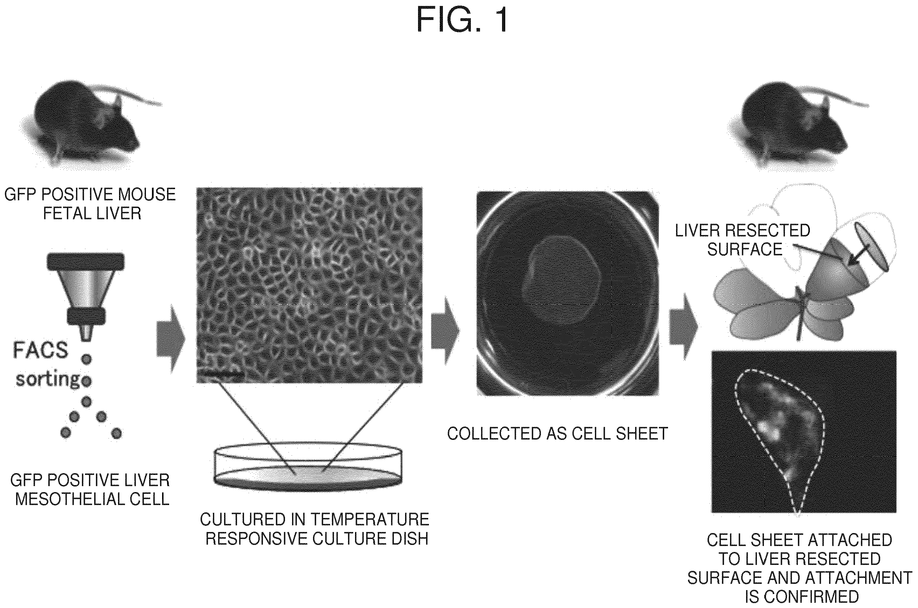

FIG. 1 schematically shows an adhesion preventing method in Examples. First, GFP positive liver mesothelial cells were isolated from a recombinant mouse expressing green fluorescent protein (GFP) using a cell sorter and cultured on a temperature responsive culture dish. A cell sheet obtained by changing the temperature was collected from the culture dish and transplanted onto the liver resected surface. Whether the sheet was attached to the liver resected surface or not was confirmed by detecting GFP. Details of the materials and methods will be explained below.

### 2. Animal

Wild type male C57BL/6J mice (10 weeks of age) were purchased from Nihon CLEA. Recombinant C57BL/6J mice expressing green fluorescent protein (GFP) were provided by Dr. Okabe of Osaka University. The age of the mouse embryos was determined by days after appearance of the vaginal plug (noon of the day when the vaginal plug appeared was considered as E0.5).

### 3. Preparation of partial hepatectomy (PPHx) mouse model

Under general anesthesia, wild type C57BL/6J mice were each placed in a spine position. The mouse was incised along a median line 2 cm above from Xyphoid inferiorly while lifting up the peritoneal so as not to damage the intraperitoneal organ. After laparotomy, the falciform ligament was separated until it reached the superior vena cava. First, the pedicle of the median lobe was tied with 4-0 silk and cut off to remove the median lobe. With an electric scalpel, the hepatic capsule around the left lobe was cauterized and the left lobe was resected at the middle thereof. During excision, the left portal veins and the left hepatic veins were ligated *en bloc* and separated.

After confirming hemostasis, the epididymis adipose tissue was lifted up to the upper part of the abdomen and placed on the resected surface of the left lobe. At the time of abdominal closure, the epididymis adipose tissue was sutured with the abdominal wall by a single stitch in order to prevent misalignment.

The mice were sacrificed 10 days or 30 days after the surgery. An adhesion percentage of each mouse was determined by dividing the adhesion length by the length of the liver resected surface.

### 4. Isolation and culture of fetal liver mesothelial cells

Fetal liver mesothelial cells were isolated from E12.5 GFP recombinant mice according to the method of Onitsuka, et al. (Onitsuka, I. et al., Gastroenterology. 138, 1525-1535(2010)). Described briefly, the fetal liver tissue was minced, isolated using Liver Digest Medium (Life Technologies), and hemolyzed in a hypotonic buffer. The cells were blocked with an anti-FcR antibody, co-stained with Anti-Mouse Podocalyxin-Biotin (MBL), and then incubated with APC-conjugated streptavidin (Invitrogen). The sample was sorted using a cell sorter "Moflo XDP" (Beckman Coulter). The sorted sample was cultured *in vitro* in a culture dish coated with type IV collagen while using MEMα medium (containing 10% fetal bovine serum (FBS), 50 nmol/L mercaptoethanol, penicillin, streptomycin, glutamine, 10 ng/mL basic fibroblast growth factor (bFGF), 10 ng/mL Oncostatin M (product of OSM), and insulin-transferrin-selenium-X) in an incubator of 37°C and 5% CO₂.

### 5. Isolation and culture of primary adult skin fibroblast

The adult fibroblasts were collected from GFP recombinant mouse skin tissue. The skin tissue was minced and treated with 0.05% trypsin and 0.5 mM EDTA at 37°C for 20 minutes. The cell suspension thus obtained was centrifuged and resuspended in a culture medium.

### 6. Isolation and culture of primary peritoneal mesothelial cells

Adult peritoneal mesothelial cells were collected from the epididymis fat of GFP recombinant mice in accordance with the method of Loureiro, et al. (Loureiro, J. et al., Nephrol Dial Transplant. 25, 1098-1108(2010)). The epididymis fat was treated at 37°C for 30 minutes with 0.05% trypsin and 0.5 mM EDTA. The cell suspension was centrifuged at 1500 rpm for 5 minutes and the resulting pellets were treated on ice for 5 minutes in a hemolysis buffer. After washing several times with PBS, the resulting peritoneal mesothelial cells were resuspended in RPMI1640 (20% FBS, penicillin, streptomycin, glutamine, and hydrocortisone (0.4 µg/mL)) and seeded in a culture dish coated with type I collagen.

### 7. Preparation of cell sheet and transplantation to PPHx mouse

The primary culture cells were each transferred to an UpCell (trade mark) plate (Cellseed Inc.) and cultured. The plate was placed at room temperature, the cells in the sheet form were collected from the culture dish, and a cell sheet was obtained.

The cell sheet was transplanted onto the liver resected surface of the mouse rightly after PPHx, followed by abdominal closure. The cell sheet engrafted only by placing on the liver resected surface. The surgery was monitored while detecting a GFP signal by 130S-GFP (product of Science eye).

### 8. AST and ALT tests

The plasma concentration of each of aspartate aminotransferase (AST) and alanine aminotransferase (ALT) was measured using a commercially available kit ("Transaminase C2-Test Wako", Wako Pure Chemical Industries).

### 9. Serum albumin test

Measurement of the serum albumin concentration was entrusted to Oriental Yeast Co., Ltd.

### 10. Histology

Frozen sections (8 µm) of the left lobe were prepared using Microm HM505E cryostat (Microm International GmbH) and mounted on APS-coated glass slides (Matsunami Glass Industry). The sections were fixed with PBS containing 4% paraformaldehyde (PFA) and stained with hematoxylin-eosin (HE).

### 11-Quantitative RT-PCR

From each of the cell sheets, total RNA was extracted using a Trizol reagent (Invitrogen) and cDNA was synthesized using High Capacity cDNA Reverse Transcription Kit (Takara Bio Inc.). Quantitative RT-PCR was performed using LightCycler 480 (product of Roche) and the following primers.
Mouse PCLP1:
TCCTTGTTGCTGCCCTCT (SEQ ID NO: 1)
CTCTGTGAGCCGTTGCTG (SEQ ID NO: 2)
Mouse HGF:
CACCCCTTGGGAGTATTGTG (SEQ ID NO: 3)
GGGACATCAGTCTCATTCACAG (SEQ ID NO: 4)
Mouse IL6:
GCTACCAAACTGGATATAATCAGGA (SEQ ID NO: 5)
CCAGGTAGCTATGGTACTCCAGAA (SEQ ID NO: 6)
Mouse Mdk:
CGCACTGGTAAAACCGAACT (SEQ ID NO: 7)
GAAGAAGCCTCGGTGCTG (SEQ ID NO: 8)
Mouse Ptn:
AGGACCTCTGCAAGCCAAA (SEQ ID NO: 9)
CACAGCTGCCAAGATGAAAAT (SEQ ID NO: 10)
Mouse β actin:
CTAAGGCCAACCGTGAAAAG (SEQ ID NO: 11)
ACCAGAGGCATACAGGGACA (SEQ ID NO: 12)
Universal Probe Library Mouse ACTB Gene Assay (Roche Inc.)

### 12. Quantification of cell size and the number of proliferating cells

After immobilization with 4% PFA-containing PBS, sections (8 mm) of the left lobe including the liver resected surface were blocked with a blocking reagent at room temperature for one hour and the resulting sections were allowed to stand overnight at 4°C in an antibody solution. 1:300 diluted rabbit anti-mouse Ki67 antibodies (product of Leica biosystems) were used as the primary antibodies, while Alexa Fluor 555 goat anti-rabbit IgGs (H+L) (product of Invitrogen) were used as the secondary antibodies. Nuclei were counterstained with Hoechst 33342 (Sigma-Aldrich). The cellular outlines were stained with Alexa Fluor 488 phalloidin (Invitrogen).

The cell size and the percentage of proliferating cells were determined using IN Cell Analyzer 2000 (GE Healthcare) in accordance with the method of Miyaoka, et al. (Miyaoka Y. et al., Curr Biol. 22, 1166-1175(2012)). Described briefly, the cell size was determined by measuring, as the outline of hepatocytes, phalloidin-stained actin surrounding the periphery of the nuclei stained with Hoechst 33342. The percentage of proliferating cells was determined by detecting and counting Ki67 positive cells while making use of staining with Hoechst 33342 and phalloidin.

Four photographs were taken for each mouse. Each photograph included from 500 to 700 hepatocytes, meaning that from 2000 to 2800 cells/mouse were analyzed in total.

### 13. Allotransplantation

PPHx mouse model was prepared using a BALB/c mouse in a manner similar to that employed above in item 3.

Fetal liver mesothelial cells were obtained from a GFP recombinant C57BL/6J mouse as described above in 4. A cell sheet was prepared as described above in item 7 and was transplanted into the PPHx mouse model.

### 14. Preparation of peritoneal adhesion model and transplantation of cell sheet

A peritoneal mesothelial cell-deficient site was prepared by detaching peritoneal mesothelial cells from the C57BL/6J mouse and a peritoneal adhesion model was established. As described above in item 7, the liver mesothelial cell sheet prepared using the liver mesothelial cells derived from the GFP recombinant C57BL/6J mouse was transplanted onto the peritoneal mesothelial cell-deficient site. In Control group, the abdomen was closed without transplanting the cell sheet.

On postoperative day 10, the adhesion was observed and the GFP signal of the peritoneal deficient-site was detected.

### 15. Statistics

The results are presented as means ± standard deviations. For the statistics, Student t-Test was used. A p value < 0.05 was considered to indicate statistical significance.

### [Results]

### 1. PPHx mouse model

A liver resected surface was prepared by the above-described method and thereby a mouse model having adhesion was prepared. The process of the liver damage after hepatectomy was similar to that of postoperative course in humans. It has therefore been confirmed that the mouse model is an animal model that reflects the human hepatectomy more (data not shown).

### 2. Autotransplantation of cell sheet to liver resected surface

### (1) Determination of adhesion percentage

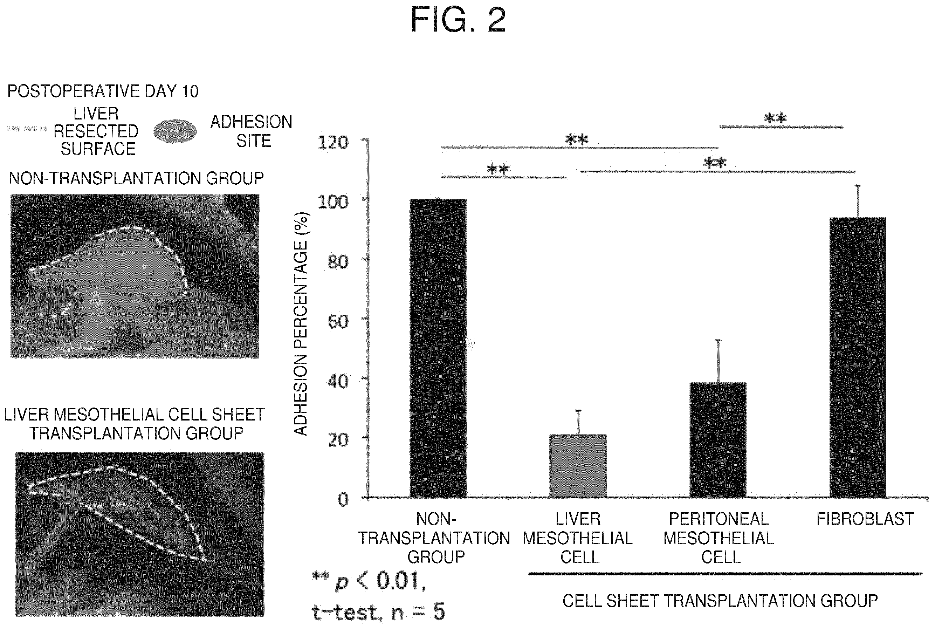

On the left side of FIG. 2, two photographs are shown: one is the PPHx mouse model to which the liver mesothelial cell sheet has been autotransplanted and the other one is a mouse to which nothing has been transplanted, each on postoperative day 10. As shown in the photographs, adhesion can be observed from the entire liver resected surface of the non-transplanted mouse, while almost no adhesion can be observed from the liver resected surface of the mouse transplanted with the liver mesothelial cell sheet. Similar results were observed even on postoperative day 30.

On the right side of FIG. 2, calculation results of an adhesion percentage are shown. Described specifically, the liver mesothelial cell sheet, the peritoneal mesothelial cell sheet, and the fibroblast cell sheet were each autotransplanted onto the liver resected surface of the PPHx mouse model and on postoperative day 10, the adhesion percentage was calculated.

Adhesion was suppressed largely in the liver mesothelial cell sheet transplantation group and the peritoneal mesothelial cell sheet transplantation group and their adhesion percentages were about 20% and about 40%, respectively. In the fibroblast cell sheet transplantation group, on the other hand, the adhesion percentage was almost 100% similar to that in the non-transplantation group.

### (2) Evaluation of liver regeneration

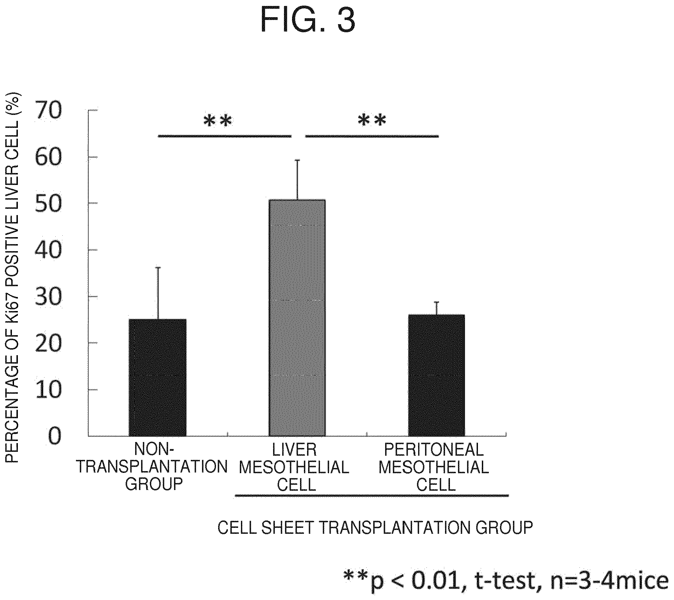

FIG. 3 shows the evaluation results of the postoperative liver regeneration in the liver mesothelial cell transplantation group, the peritoneal mesothelial cell transplantation group, and non-transplantation group, with the percentage of Ki67 positive hepatocytes as an indicator. In the liver mesothelial cell sheet transplantation group, the percentage of Ki67 positive hepatocytes was almost twice as much as that of the non-transplantation group, revealing that the cell proliferation was promoted.

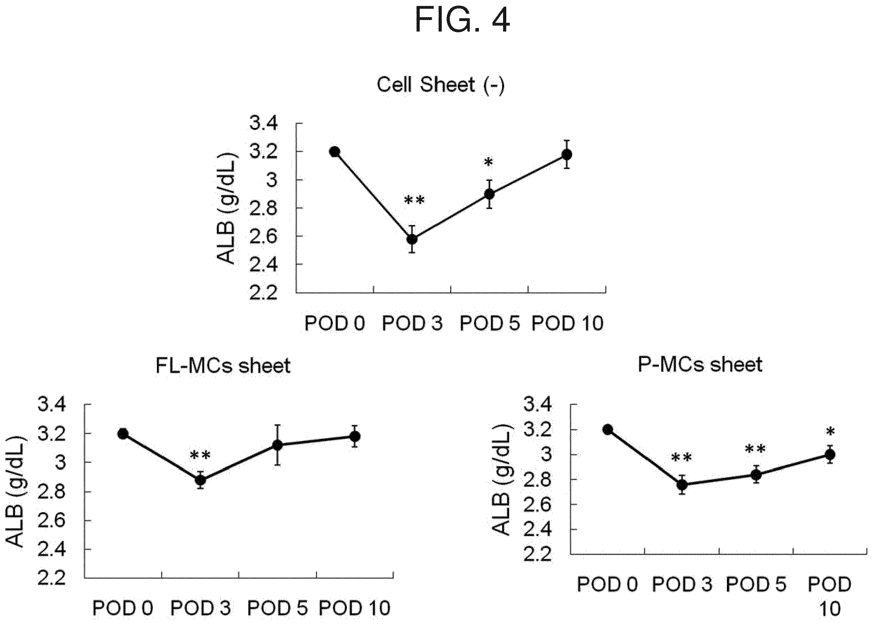

FIG. 4 shows the evaluation results of postoperative liver regeneration in the liver mesothelial cell transplantation group, the peritoneal mesothelial cell transplantation group, and non-transplantation group, with the serum albumin as an indicator. It has been confirmed that in the liver mesothelial cell sheet transplantation group, postoperative recovery of the serum albumin was promoted. Early recovery of liver function is very desirable since it is known to contribute largely to the avoidance of postoperative liver failure.

### 3. Allotransplantation of cell sheet to liver resected surface

### (1) Determination of adhesion percentage

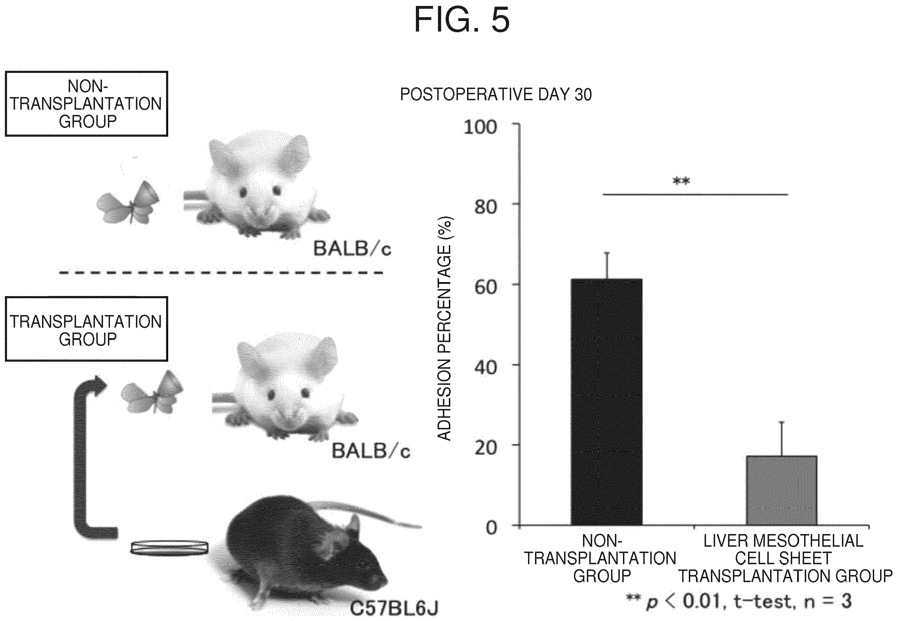

FIG. 5 shows the results of an adhesion percentage measured by transplanting the cell sheet prepared using the liver mesothelial cells derived from a C57BL/6J mouse onto the BALB/c PPHx mouse model. Similar to the adhesion percentage of the autotransplanted sheet, a drastic reduction in adhesion percentage was observed in the liver mesothelial cell sheet transplantation group.

### (2) Evaluation of liver regeneration

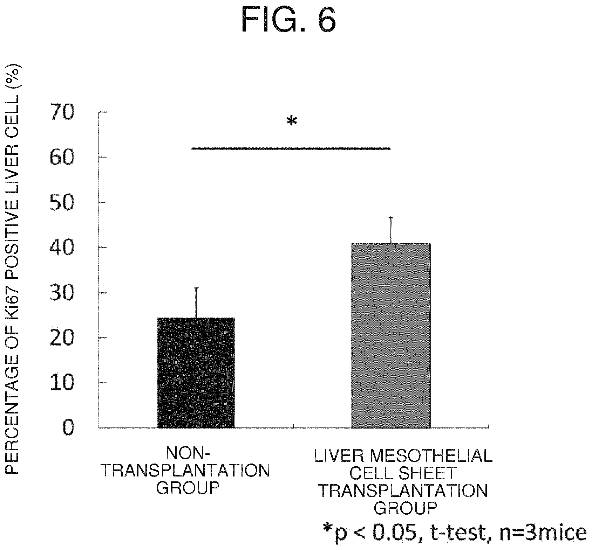

FIG. 6 shows the evaluation results of postoperative liver regeneration with the percentage of Ki67 positive hepatocytes as an indicator. Similar to the liver regeneration caused by autotransplantation, the percentage of the Ki67 positive hepatocytes in the liver mesothelial cell sheet transplantation group was almost twice as much as that in the non-transplantation group, revealing the promotion of cell proliferation.

### 4. Transplantation of liver mesothelial cell sheet into peritoneal adhesion model

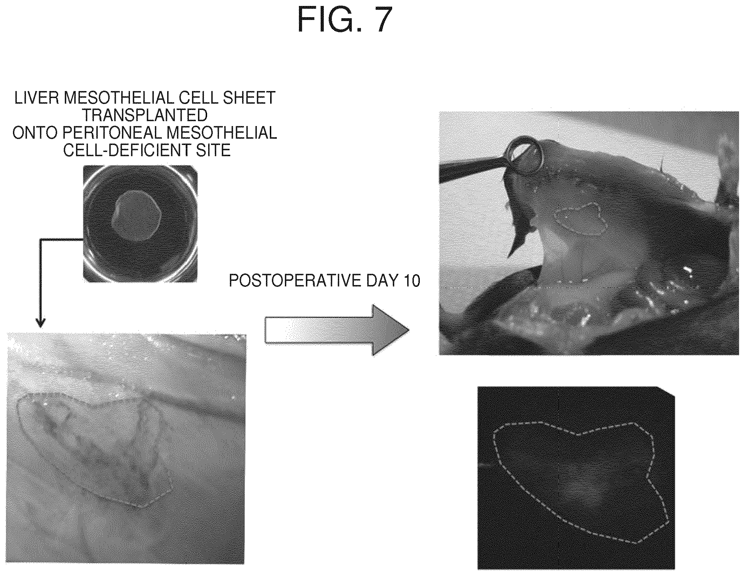

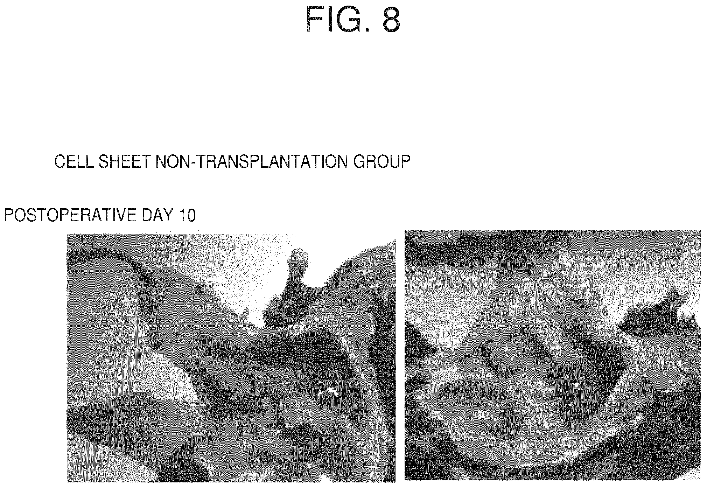

FIG. 7 shows the observation results of the peritoneal adhesion model that transplanted with the liver mesothelial cell sheet and subjected to laparotomy 10 days after transplantation. FIG. 8 shows the observation results of the model subjected to laparotomy after 10 days without transplanting the liver mesothelial cell sheet. In the non-transplantation group, intestines in the abdominal cavity adhered firmly to the peritoneal wall-deficient site, while in the liver mesothelial cell transplantation group, adhesion was markedly suppressed. The GFP signal derived from the liver mesothelial cell sheet was observed at the peritoneal wall-deficient site, suggesting that the liver mesothelial cells engrafted and prevented intraperitoneal adhesion.

### [Sequence listing free text]

SEQ ID NOS: 1 and 2 represent primers used for mouse PCLP1.
SEQ ID NOS: 3 and 4 represent primers used for mouse HGF.
SEQ ID NOS: 5 and 6 represent primers used for mouse IL6.
SEQ ID NOS: 7 and 8 represent primers used for mouse Mdk.
SEQ ID NOS: 9 and 10 represent primers used for mouse Ptn.
SEQ ID NOS: 11 and 12 represent primers used for mouse β actin.

## Claims

1. A material for use in a method of preventing adhesion of a resected surface after partial hepatectomy, wherein the material comprises a cell sheet comprising liver mesothelial cells.

2. The material for use according to Claim 1, wherein the mesothelial cells are cells derived from the same individual as the subject of the adhesion prevention.

3. The material for use according to Claim 1, wherein the mesothelial cells are cells derived from an individual different from the subject of the adhesion prevention.

4. The material for use according to any one of Claims 1 to 3, wherein the mesothelial cells are cells derived from an individual from fetal to infant stages.

5. The material for use according to any one of Claims 1 to 4, wherein the material is placed on the resected surface.

6. The material for use according to any one of the preceding claims, wherein the material is further for use in promoting liver regeneration.

7. The material for use according to any one of Claims 1 to 6, which does not comprise a support but consists only of the cell sheet.

## Patentansprüche

1. Material zur Verwendung in einem Verfahren zur Prävention des Anhaftens einer resezierten Oberfläche nach einer Teilhepatektomie, wobei das Material eine Lebermesothelzellen umfassende Zelllage umfasst.

2. Material zur Verwendung nach Anspruch 1, wobei die Mesothelzellen von demselben Individuum, welches auch der Adhäsionsprävention unterzogen wird, stammende Zellen sind.

3. Material zur Verwendung nach Anspruch 1, wobei die Mesothelzellen von einem anderen Individuum als jenem stammen, welches der Adhäsionsprävention unterzogen wird.

4. Material zur Verwendung nach einem der Ansprüche 1 bis 3, wobei die Mesothelzellen von einem Individuum in der Fötus- bis Kleinkindphase stammende Zellen sind.

5. Material zur Verwendung nach einem der Ansprüche 1 bis 4, wobei das Material auf der resezierten Oberfläche angeordnet wird.

6. Material nach einem der vorangegangenen Ansprüche, wobei das Material weiters zur Verwendung zur Unterstützung der Leberregeneration dient.

7. Material zur Verwendung nach einem der Ansprüche 1 bis 6, das keinen Träger umfasst, sondern ausschließlich aus der Zelllage besteht.

## Revendications

1. Matériau destiné à être utilisé dans un procédé de prévention d'adhérence d'une surface réséquée après une hépatectomie partielle, dans lequel le matériau comprend une feuille de cellules comprenant des cellules mésothéliales de foie.

2. Matériau destiné à être utilisé selon la revendication 1, dans lequel les cellules mésothéliales sont des cellules dérivées du même individu que le sujet de la prévention d'adhérence.

3. Matériau destiné à être utilisé selon la revendication 1, dans lequel les cellules mésothéliales sont des cellules dérivées d'un individu différent du sujet de la prévention d'adhérence.

4. Matériau destiné à être utilisé selon l'une quelconque des revendications 1 à 3, dans lequel les cellules mésothéliales sont des cellules dérivées d'un individu compris entre les stades de foetus et de nourrisson.

5. Matériau destiné à être utilisé selon l'une quelconque des revendications 1 à 4, dans lequel le matériau est placé sur la surface réséquée.

6. Matériau destiné à être utilisé selon l'une quelconque des revendications précédentes, dans lequel le matériau est en outre destiné à être utilisé pour favoriser une régénération de foie.

7. Matériau destiné à être utilisé selon l'une quelconque des revendications 1 à 6, qui ne comprend pas de support mais qui est constitué uniquement de la feuille de cellules.
